# EUROPEAN PATENT APPLICATION

(11) **EP 2 476 454 A2**
(43) Date of publication of application: **18.07.2012**
(21) Application number: 10815597.9
(22) Date of filing: 07.09.2010
(51) Int. Cl.: A61M 35/00, A61M 37/00

(54) **APPARATUS FOR PRECISELY REGULATING A LIQUID MEDICINE INJECTION DOSE**

(30) Priority: 09.09.2009 KR 20090084898
(71) Applicant: Park, Hyo Nam, Suwon-si, Gyeonggi-do 441-884 (KR)
(72) Inventor: Park, Hyo Nam, Suwon-si, Gyeonggi-do 441-884 (KR)
(74) Representative: Kuhnen & Wacker
(86) International application number: PCT/KR2010/006064
(87) International publication number: WO 2011/031052

(57) **Abstract**

Disclosed therein is an apparatus for precisely regulating a liquid medicine injection dose. The apparatus according to one embodiment of the present invention comprises an adjustment knob, a gate seal, and a body. The apparatus injects liquid medicine regulated in a stable, precise, and minute manner. The apparatus for precisely regulating a liquid medicine injection dose has a reduced number of components, and thus can be easily manufactured at a low cost.

## Description

### [Technical Field]

The present invention relates to an apparatus for precisely regulating a liquid medicine injection dose, and more particularly, to an apparatus for precisely regulating a liquid medicine injection dose, which can minutely regulate a dose of liquid medicine injected into a patient's body so that the liquid medicine can be injected stably and correctly.

### [Background Art]

Patients, who go into hospitals or are depressed in digestive functions, receive a medication, so called a Ringer's solution, such as nutritional supplements, dextrose, and so on. In this instance, the Ringer's solution is injected to the patient through a flow rate regulator for a liquid medicine, which we can see in hospitals. The flow rate regulator for the liquid medicine regulates an amount of the liquid medicine injected from a Ringer bottle or pack into the patient's blood vessel in a state where an end of a hose connected to the Ringer bottle or pack is connected to a needle inserted into the patient's blood vessel.

Presently, Ringer devices generally used in medical institutions adopt a method of securing position energy by lifting up the Ringer pack or bottle containing the Ringer's solution higher than the needle using gravity and injecting the Ringer's solution into the patient's body using a pressure difference, and are divided into two kinds.

First, there is a flow rate regulating method of mounting the hose connected with the needle inside the flow rate regulator, and pressing the hose by moving a press roller moving inside an inclined groove to thereby change a section of the hose where the liquid medicine flows, so that the flow rate of the liquid medicine can be regulated through contraction and expansion of the sectional area of the hose.

Such a method is simple in manufacturing and inexpensive, but because the press roller for pressing the hose is gradually moved from the original pressing position by a restoring force of the hose made of a soft synthetic resin or rubber, so that the sectional area of the hose is expanded and an excess amount of the liquid medicine may be injected into the patient's body, whereby it may cause emergency circumstances. Moreover, there are different margin of errors in flow rate of the liquid medicine according to users' skill levels and it is difficult to correctly and minutely regulate the flow rate of the liquid medicine.

Because of the above problems, guardians or nurses must observe the flow rate of the liquid medicine very carefully, and in the case that the liquid medicine is continuously injected, there is inconvenience that the user must regulate the flow rate of the liquid medicine again and it is difficult to set the flow rate of the liquid medicine in the same way as it was when the Ringer bottle or pack is replaced.

Second, there is a flow rate regulating method of forming a fine flow path inside a flow rate regulating valve of the flow rate regulator to regulate the flow rate of the liquid medicine using a flow resistance of a fluid. The second flow rate regulating method can regulate the flow rate more correctly than the first regulating method.

The second regulating method has an improved precision in comparison with the first regulating method, but has several inconveniences in use and inadequate points in safety.

That is, in the case that the user manipulates a manipulation valve to regulate a flow rate, because the manipulation valve rotating by the user's hand and the flow rate regulating valve for rotating the flow path are directly connected with each other, when the user rotates the manipulation valve in a stationary state, since it requires power more than a force of static friction, it is difficult to minutely regulate gradations. Furthermore, when the manipulation valve is manipulated, it requires a relatively great rotational force by the user. Additionally, it is inconvenient to completely discharge air which may remain in the hose because the manipulation valve must be perfectly rotated, and the manipulation valve is rotated by error even though the flow rate is set, and hence, it may cause medical accidents, for instance, the flow rate may be changed. In addition, the flow rate regulator requires a great deal of components, is complicated in structure, frequently causes troubles, and is very expensive.

### [Disclosure]

### [Technical Problem]

Accordingly, the present invention has been made in an effort to solve the above-mentioned problems occurring in the prior arts, and it is an object of the present invention to provide an apparatus for precisely regulating a liquid medicine injection dose, which can precisely regulate the dose of a liquid medicine in safety to thereby correctly inject the liquid medicine to a patient's body and is easy to manufacture and can reduce manufacturing costs by reducing the number of components.

### [Technical Solution]

To achieve the above objects, the present invention provides an apparatus for precisely regulating a liquid medicine injection dose including an adjustment knob, a gate seal, and a body.

The adjustment knob includes: a first body having a flow path that is formed at a predetermined interval from the center of the bottom face thereof and has a depth that is different from a start point to an end point thereof; a second body being larger in diameter than the second body and formed integrally with the upper portion of the first body to thereby rotate with the first body; a third body being smaller in diameter than the first body and formed integrally with the lower portion of the first body to thereby rotate with the first body; and a first central hole being formed at the centers of the first body, the second body, and the third body.

The gate seal of a cylindrical shape being surrounds the first body and the third body, which are inserted into the gate seal, and includes: an inflow hole formed in the wall surface of one side thereof for injecting the liquid medicine into the flow path; an outflow hole formed in the wall surface of the other side thereof for discharging the liquid medicine discharged from the flow path; a tiered portion formed along the inner wall surface and having a through hole that is formed in the upper face of the tiered portion, is in direct contact with a bottom surface of the first body and connected to the outflow hole, so that an amount of the liquid medicine is regulated by means of rotation of the flow path and the liquid medicine is discharged through the through hole; and a second central hole formed at the center of the tiered portion in correspondence to the first central hole.

The body, to which the gate seal is inserted and fixed, includes: an insertion portion having a projection pole formed at the center of the bottom face and inserted into the first central hole and the second central hole to combine the adjustment knob with the gate seal, an inlet formed on one side of the wall surface for injecting the liquid medicine into the inflow hole, and an outlet formed on the other side of the wall surface in correspondence to the outflow hole; and a grip portion being elongated from the outer wall surface of the insertion portion in one direction.

The first body of the adjustment knob is formed in a cylindrical shape, and the flow path is formed in a concentric circle relative to the center of the bottom surface of the first body and comprises: a first portion which is gradually deepened from the start point to the end point; and a purge portion which is wider and deeper than the first portion but shorter than the first portion.

The second body of the adjustment knob includes: a first rotational gear portion that is formed integrally to the upper portion of the first body, is larger in diameter than the first body, and has saw teeth formed on the circumferential surface thereof; and a second rotational gear portion that is formed integrally to the upper portion of the first rotational gear portion, is larger in diameter than the first rotational gear portion, and has saw teeth formed on the circumferential surface thereof and gradations formed on the upper surface for indicating the amount of the liquid medicine.

The first rotational gear portion includes: a first projection formed on the bottom surface thereof to prevent the liquid medicine from leaking out by pressing a portion which is in contact with the upper face of the wall surface of the gate seal; and a stop portion formed on the outer face of the first projection of the bottom surface of the first rotational gear portion and on the opposite side of the purge portion for preventing the second body rotating along the outer wall surface of the insertion portion of the body from rotating more.

A purge hole is formed in one side of the bottom face of the stop portion, and a stop projection is protrudingly formed on the upper portion of the inlet of the outer wall surface of the body, so that the stop portion is caught to the stop projection and the second body is not rotated anymore. A purge projection is formed on the outer wall surface of the body spaced apart at a predetermined interval from the stop projection and coupled to the purge hole, so that the user can recognize the purge state that the amount of the liquid medicine is discharged to the maximum, and the purge projection is caught to the purge hole when the stop portion is caught to the stop projection.

A second projection portion is formed on the bottom surface of the third body for pressurizing a portion, which is in contact with the tiered portion of the gate seal to thereby prevent the liquid medicine from leaking. A plurality of coupling poles are formed on the inner wall surface of the third body in which the first central hole is formed and respectively have coupling projections that respectively protrude at ends of the coupling poles toward the central direction, the coupling poles being upwardly projected in a cylindrical shape.

The tiered portion of the gate seal includes: a first tier formed along the inner wall surface of the gate seal and corresponding to the bottom surface of the first body; and a second tier formed beneath the first tier and along the inner wall surface of the first tier and corresponding to the bottom surface of the third body, and the second tier has a diameter smaller than that of the first tie.

A projection face having the through hole is formed on the upper face of the first tier, and protrudes more upwardly than the remaining portion of the upper face of the first tier and being in direct contact with the bottom surface of the first body. A space is formed between the remaining portion of the upper face of the first tier excepting the projection face and the bottom surface of the first body in such a fashion that the space is filled with the liquid medicine, and the through hole is formed vertically downward and is connected with the outflow hole at the bottom face of the gate seal.

The gate seal includes: a surrounding portion that is formed on the bottom face of the gate seal in such a way as to be depressed upwardly and surrounds the second central hole; and at least one fixing hole that is formed in the outward direction of the bottom face from the surrounding portion.

The insertion portion of the body includes: a fixing projection that is formed on the bottom face of the insertion portion and is inserted and fixed into the fixing hole of the bottom face of the gate seal; and a third projection portion that surrounds the projection pole and pressurizes the surrounding portion of the bottom face of the gate seal to fix the gate seal, and a stopper is mounted on the upper face of the grip portion and inserted between the saw teeth of the first rotational gear portion to fix the adjustment knob so that the adjustment knob is not rotated.

The stopper includes: an upper body that has a stopper handgrip inserted between the saw teeth of the first rotational gear portion and a detachably mounted portion formed beneath the stopper handgrip; and a seating recess portion that is formed integrally with the grip portion and moves back and forth in a state where the detachably mounted portion is forcedly inserted into the seating recess portion.

The tiered portion of the gate seal includes: a first tier formed along the inner wall surface of the gate seal and corresponding to the bottom surface of the first body; a second tier formed beneath the first tier and along the inner wall surface of the first tier and corresponding to the bottom surface of the third body, the second tier having a diameter smaller than that of the first tier, and a flow path groove formed along the upper face of the first tier in such a way as to correspond to the flow path of the bottom surface of the first body and to be filled with the liquid medicine. The through hole is formed between a start point and an end point of the flow path groove in such a fashion that the through hole is spaced apart from the start point and the end point of the flow path groove at a predetermined interval, and the liquid medicine filling the flow path groove and the flow path is discharged through the through hole when the upper face of the first tier gets in direct contact with the bottom surface of the first body, the flow path groove is connected with the inflow hole, and the through hole is formed vertically downward and connected with the outflow hole on the bottom face of the gate seal.

### [Advantageous Effects]

The apparatus for precisely regulating the liquid medicine injection dose according to the present invention has following advantages.

First, the conventional regulating apparatuses adopt the inlet control method to regulate an introduced amount of the liquid medicine by matching the inlet to the flow path, and hence, it is difficult to precisely regulate the flow rate of the liquid medicine because the resistance is strong from a standpoint of the liquid since the whole length of the flow path ranging from inflow to discharge of the liquid medicine. However, the regulating apparatus according to the present invention adopting the outlet control method that regulates the discharged amount of the liquid medicine while the through hole of the projection face connected with the outflow hole moves on the surface of the flow path can precisely regulate the flow rate of the liquid medicine because the resistance is weak from a standpoint of the liquid.

Second, because the flow path is formed on the bottom face of the first body, there is no division line formed on a mold during an injection-molding, and hence, the flow path can be injection-molded in a uniform size. Because the regulating apparatus according to the present invention has just four components, namely, the adjustment knob, the gate seal, the body, and the stopper, compared with the conventional regulating apparatuses, it can greatly reduce the number of components to thereby reduce the manufacturing costs by decreasing a mold price, an injection-molding price, and assembling work expenses.

Third, the regulating apparatus according to the present invention can rapidly discharge all of air contained in the hose and rapidly perform a purge function to fill the hose with the liquid medicine at the same time when a reverse rotation is carried out at the start point of the flow path.

Fourth, the stopper is assembled by one touch, and the stopper may has a through hole formed on the upper portion thereof so that the user can see gradation of the present flow rate of the liquid medicine with eyes, and hence, the present invention does not need any indication lines printed for indicating the present gradation.

### [Description of Drawings]

The preferred embodiment of the present invention will now be described by way of example with reference to the accompanying diagrammatic drawings.
FIG. 1 is a perspective view showing the entire structure of an apparatus for precisely regulating a liquid medicine injection dose according to a preferred embodiment of the present invention.
FIG. 2 is an exploded perspective view of the regulating apparatus of FIG.1.
FIG. 3 is a side view of an adjustment knob.
FIG. 4 is a perspective view of the adjustment knob viewed from the bottom.
FIG. 5 is a perspective view of the adjustment knob viewed from the top.
FIG. 6 is a perspective view of a gate seal viewed from the top.
FIG. 7 is a perspective view of the gate seal viewed from the bottom.
FIG. 8 is a bottom view of the gate seal.
FIG. 9 is a perspective view of a body.
FIG. 10 is a right side view showing a state where the adjustment knob, the gate seal, and the body are combined with each other.
FIG. 11 is a view for explaining another structure of the gate seal.

### [Mode for Invention]

The above and other advantages of the present invention and goals achieved by the preferred embodiment of the present invention will be apparent from the following detailed description of the preferred embodiment of the invention in conjunction with the accompanying drawings.

Reference will be now made in detail to a bicycle tire 1 of the present invention with reference to the attached drawings. In the drawings, the same reference numerals designate the same components.

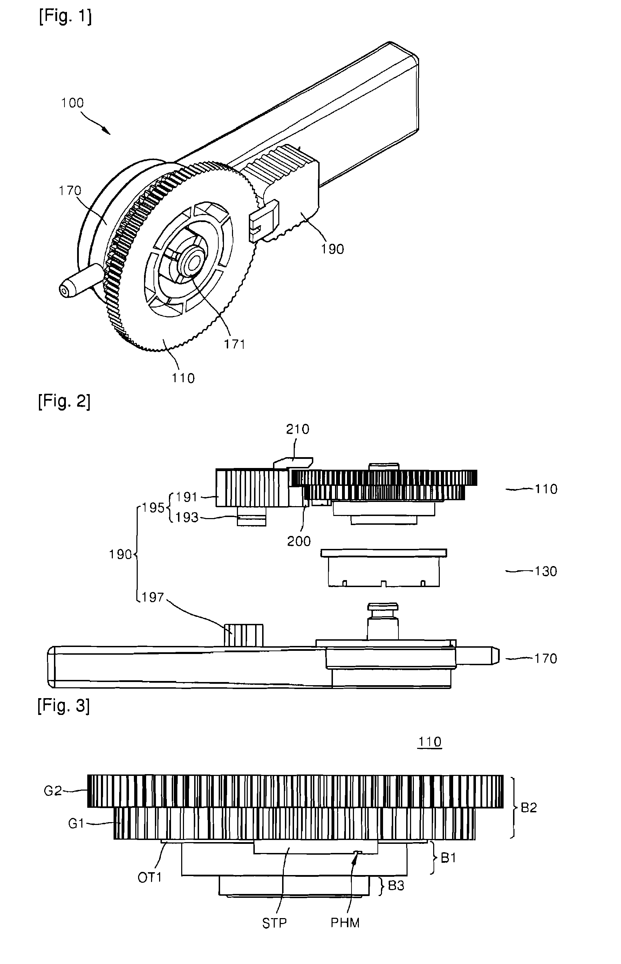

FIG. 1 is a perspective view showing the entire structure of an apparatus for precisely regulating a liquid medicine injection dose according to a preferred embodiment of the present invention, and FIG. 2 is an exploded perspective view of the regulating apparatus of FIG.1.

Referring to FIGS. 1 and 2, the apparatus 100 for precisely regulating a liquid medicine injection dose according to the preferred embodiment of the present invention includes an adjustment knob 110, a gate seal 130, and a body 170.

The gate seal 130 is inserted into the body 170, the adjustment knob 110 is inserted into the gate seal 130, and a liquid medicine introduced through an inlet 175 is discharged through an outlet 173 while an amount of the liquid medicine is adjusted by means of rotation of the adjustment knob 110.

As shown in FIG. 2, the regulating apparatus 100 is easy in manufacturing and trouble-free and can reduce manufacturing costs because it includes just four components.

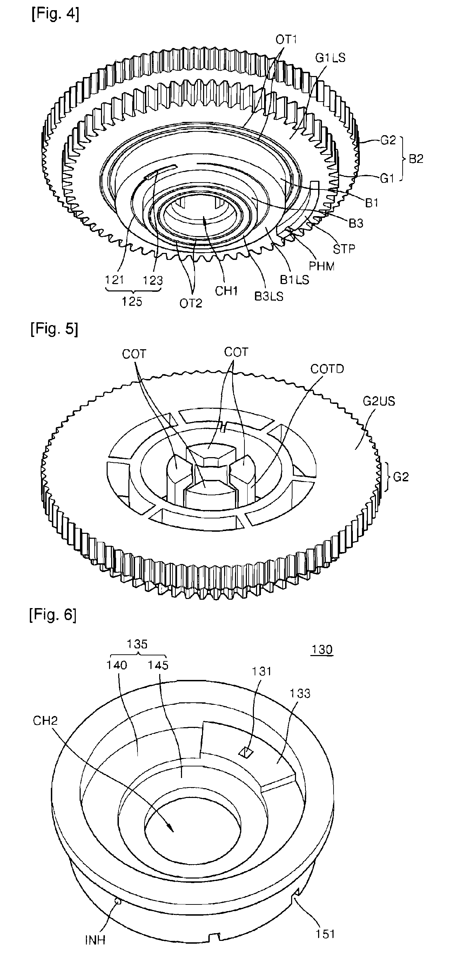

FIG. 3 is a side view of an adjustment knob, FIG. 4 is a perspective view of the adjustment knob viewed from the bottom, and FIG. 5 is a perspective view of the adjustment knob viewed from the top.

Referring to FIGS. 3 to 5, the adjustment knob 110 includes a first body B1, a second body B2, and a third body B3.

The first body B1 has a flow path 125 formed at a predetermined interval from the center of the bottom face thereof and has a depth that is different from a start point to an end point thereof. The second body B2 is larger in diameter than the second body B2, and is formed integrally with the upper portion of the first body B1 to thereby rotate with the first body B1. The third body B3 is smaller in diameter than the first body B1, and is formed integrally with the lower portion of the first body B1 to thereby rotate with the first body B1. Moreover, a first central hole CH1 is formed at the centers of the first body B1, the second body B2, and the third body B3.

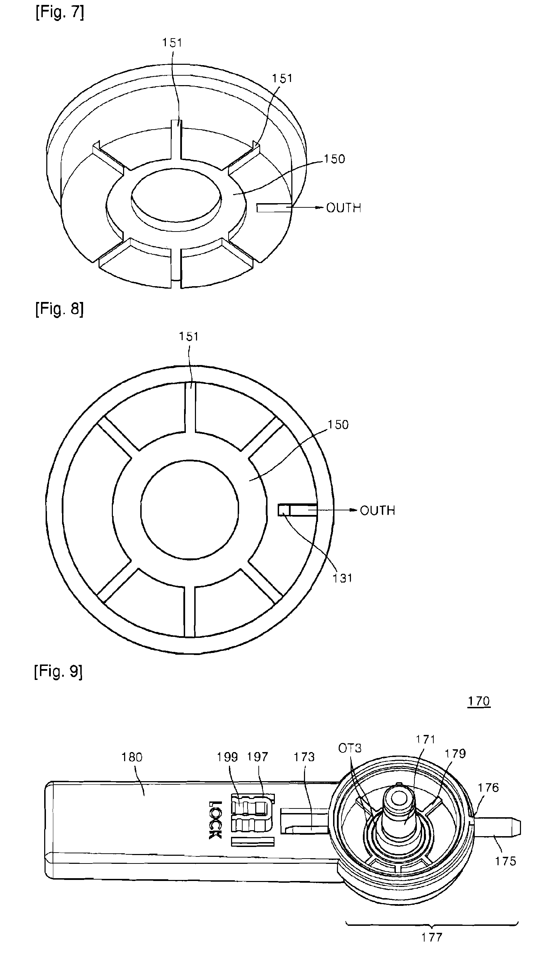

FIG. 6 is a perspective view of the gate seal viewed from the top, FIG. 7 is a perspective view of the gate seal viewed from the bottom, and FIG. 8 is a bottom view of the gate seal.

Referring to FIGS. 6 to 8, the gate seal 130 surrounds the first body B1 and the third body B3, which are inserted into the gate seal 130, and includes: an inflow hole INH formed in the wall surface of one side thereof for injecting the liquid medicine into the flow path 125; and an outflow hole OUTH formed in the wall surface of the other side thereof for discharging the liquid medicine discharged from the flow path 125.

The upper face of a tiered portion 135 formed along the inner wall surface is in direct contact with a bottom surface B1LS of the first body B1 and a through hole 131 connected to the outflow hole OUTH is formed on the upper face of the tiered portion 135, so that an amount of the liquid medicine is regulated by means of rotation of the flow path 125 and the liquid medicine is discharged through the through hole 131.

A second central hole CH2 corresponding to the first central hole CH1 is formed at the center of the tiered portion 135 and is in a cylindrical shape.

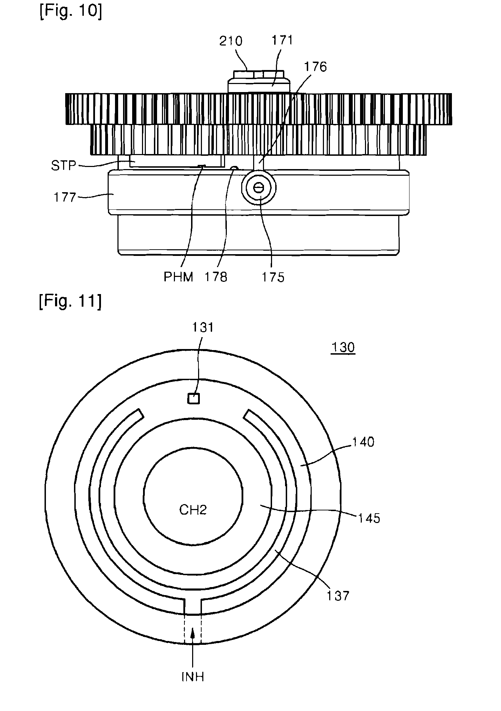

FIG. 9 is a perspective view of the body, and FIG. 10 is a right side view showing a state where the adjustment knob, the gate seal, and the body are combined with each other.

Referring to FIGS. 9 and 10, the body 170 includes an insertion portion 177 and a grip portion 180.

The gate seal 130 is inserted and fixed into the insertion portion 177, and the insertion portion 177 includes: a projection pole 171 formed at the center of the bottom face and inserted into the first central hole CH1 and the second central hole CH2 to combine the adjustment knob 110 with the gate seal 130; an inlet 175 formed on one side of the wall surface for injecting the liquid medicine into the inflow hole INH; and an outlet 173 formed on the other side of the wall surface in correspondence to the outflow hole OUTH. The grip portion 180 is elongated from the outer wall surface of the insertion portion 177 in one direction.

Hereinafter, referring to FIGS. 1 to 10, structure and operation of the apparatus 100 for precisely regulating the liquid medicine injection dose according to the preferred embodiment of the present invention will be described in detail.

The first body B1 of the adjustment knob 10 is formed in the cylindrical shape, and the flow path 125 is formed in a concentric circle relative to the center of the bottom surface B1LS of the first body B1 and includes: a first portion 121 which is gradually deepened from the start point to the end point; and a purge portion 123 which is wider and deeper than the first portion 121 but shorter than the first portion 121.

As shown in FIG. 4, the bottom surface B1LS of the first body B1 is formed in a cylindrical shape, and the flow path 125 is in the form of a circle along the circumference of the cylindrical shape. The start point and the end point of the flow path 125 are not in contact with each other but spaced apart from each other at a predetermined interval. The first portion 121 is gradually deepened toward the purge portion 123 and can regulate a flow rate of the liquid medicine according to the rotation of the first body B1. Furthermore, the purge portion 123 can allow a large amount of liquid medicine to flow at once because the purge portion 123 is bigger and deeper than the first portion 121, and it is called a 'purge function'.

The second body B2 of the adjustment knob 110 includes: a first rotational gear portion G1 that is formed integrally to the upper portion of the first body B1, is larger in diameter than the first body B1, and has saw teeth formed on the circumferential surface thereof; and a second rotational gear portion G2 that is formed integrally to the upper portion of the first rotational gear portion G1, is larger in diameter than the first rotational gear portion G1, and has saw teeth formed on the circumferential surface thereof and gradations formed on the upper surface G2US for indicating the amount of the liquid medicine.

The second rotational gear portion G2 is a portion that a user can rotate with the hand, and the saw teeth formed on the circumferential surface thereof produce friction with the user's finger. Not shown in FIG. 5, the gradations are formed on the upper surface G2US of the second rotational gear portion G2.

The first rotational gear portion G1 includes: a first projection OT1 formed on the bottom surface G1LS of the first rotational gear portion G1 to prevent the liquid medicine from leaking out by pressing a portion which is in contact with the upper face of the wall surface of the gate seal 130; and a stop portion STP formed on the outer face of the first projection OT1 of the bottom surface G1LS of the first rotational gear portion G1, namely, on the opposite side of the purge portion 123, for preventing the second body B2 rotating along the outer wall surface of the insertion portion 177 of the body 170 from rotating more.

A stopper 190 is inserted into the saw teeth of the first rotational gear portion G1 in order to fix the adjustment knob 110, and hence, the adjustment knob 110 is not rotated anymore. When the first projection portion OT1 pressurizes the upper face of the wall surface of the gate seal 130, because the gate seal 130 is made of rubber, the pressurized portion of the upper face of the gate seal 130 is pressed, but on the contrary, the remaining portion of the upper face is projected. Accordingly, it can prevent the liquid medicine from being discharged between the gate seal 130 and the bottom surface G1LS of the first rotational gear portion G1.

Referring to FIG. 10, a purge hole PHM is formed in one side of the bottom face of the stop portion STP, and a stop projection 176 is protrudingly formed on the upper portion of the inlet 175 of the outer wall surface of the body 170, so that the stop portion STP is caught to the stop projection 176 and the second body B2 is not rotated anymore.

The adjustment knob 110 is rotated to regulate the amount of the liquid medicine according to the gradations (not shown) in such a way as to gradually increase the amount of the liquid medicine from the minimum amount of the liquid medicine. Moreover, when the adjustment knob 110 is rotated a little in the opposite direction, the purge portion 123 meets the through hole 131 to carry out the purge function to discharge air out from the hose connected to the outlet 173 and fill the hose with the liquid medicine at once.

However, when the adjustment knob 110 that is rotated to the purge portion 123 is continuously rotated in the same way, the adjustment knob 110 is returned to the start point of the first portion 121 of the flow path 125, and hence, the stop portion STP is formed on the opposite side of the purge portion 123 to prevent the adjustment knob 110 from rotating one a complete round.

Furthermore, a purge projection 178 coupled to the purge hole PHM is formed on the outer wall surface of the body 170 spaced apart from the stop projection 176 at a predetermined interval, so that the user can recognize the purge state that the amount of the liquid medicine is discharged to the maximum.

As shown in FIG. 10, an interval between an end of the stop portion STP and the purge hole PHM is the same as an interval between the stop projection 176 and the purge projection 178. Accordingly, when the stop portion STP is caught to the stop projection 176, the purge projection 178 is caught to the purge hole PHM with "click". That is, when the user make the adjustment knob 110 in a purge state while referring the gradations of the upper surface G2US of the second rotational gear portion G2 of the adjustment knob 110, the purge projection 178 is caught to the purge hole PHM, so that the user can check the purge state with eyes and feel the purge state with the hands. Additionally, when the gradations are fit to the purge state, in order to prevent the adjustment knob 110 from rotating more, at the same time, the stop portion STP is caught to the stop projection 176.

A second projection portion OT2 is formed on the bottom surface B3LS of the third body B3 for pressurizing a portion, which is in contact with the tiered portion 135 of the gate seal 130 to thereby prevent the liquid medicine from leaking.

In other words, it will be described later, but the bottom surface B3LS of the third body B3 is in contact with a second tier 145 of the tiered portion 135 of the gate seal 130, and a space, which will be filled with the liquid medicine, is formed between a first tier 140 and the bottom surface B1LS of the first body B1. The second projection OT2 formed on the bottom surface B3LS of the third body B3 pressurizes the second tier 145 of the gate seal 130, so that the liquid medicine filling the space does not leak out between the second projection portion OT2 and the second tier 145 but leaks out just through the through hole 131 of a projection face 133 of the first tier 140.

A plurality of coupling poles COTD are formed on the inner wall surface of the third body B3 in which the first central hole CH1 is formed, respectively have coupling projections COT that respectively protrude at ends of the coupling poles COTD toward the central direction, and are upwardly projected in a cylindrical shape.

As shown in FIG. 5, the coupling poles COTD are formed upwardly from the inner wall surface of the third body B3 having the first central hole CH1 to a height that is parallel to the upper surface S2US of the second rotational gear portion G2 of the adjustment knob 110. Moreover, the projection pole 171 of the body 170 is inserted and fit between the coupling projections COT shown in FIG. 5.

As described above, the adjustment knob 110 includes the first body B1, the second body B2 and the third body B3 that are formed integrally with one another and rotate together, the flow path 125, and the gradations for indicating the amount of the liquid medicine. So, when the first body B1 and the third body B3 are inserted into the gate seal 130, the regulating apparatus can regulate the amount of the liquid medicine and discharge the liquid medicine while the user rotates the adjustment knob 110.

Referring to FIG. 6, the tiered portion 135 of the gate seal 130 includes: the first tier 140 formed along the inner wall surface of the gate seal 130 and corresponding to the bottom surface B1LS of the first body B1; and the second tier 145 formed beneath the first tier 140 and along the inner wall surface of the first tier 140 and corresponding to the bottom surface B3LS of the third body B3. A diameter of the second tier 145 is smaller than that of the first tier 140. The second central hole CH2 is formed at the center of the first tier 140 and the second tier 145.

The projection face 133 having the through hole 131 is formed on the upper face of the first tier 140, and in this instance, the projection face 133 projects more upwardly than the remaining portion of the upper face of the first tier 140 and is in direct contact with the bottom surface B1LS of the first body B1, so that the space is formed between the remaining portion of the upper face of the first tier 140 excepting the projection face 133 and the bottom surface B1LS of the first body B1 and is filled with the liquid medicine. The size of the projection face 133 may be varied, but preferably, the projection face 133 is formed within a range of about 60-degree angle from the center of the gate seal 130.

Furthermore, the through hole 131 formed in the projection face 133 is formed vertically downward, and is connected with the outflow hole OUTH at the bottom face of the gate seal 130. It is illustrated in FIG. 8.

As shown in FIG. 6, because the projection face 133 of the first tier 140 is in contact with the bottom surface B1LS of the first body Bl, it becomes in direct contact with the flow path 125. Additionally, because the remaining portion of the first tier 140 excepting the projection face 133 is not in direct contact with the bottom surface B1LS of the first body B1 but is spaced apart from the bottom surface B1LS as long as the height of the projection face 133, the space is naturally formed. Because the inflow hole INH of the gate seal 130 is formed in the wall surface of the gate seal 130 higher than the first tier 140, when the liquid medicine is injected through the inflow hole INH, the space between the first tier 140 and the bottom surface B1LS of the first body B1 is filled with the liquid medicine and the flow path 125 is also filled with the liquid medicine.

In addition, when the adjustment knob 110 is rotated, the projection face 133 gets in contact with the flow path 125, and the liquid medicine passes the through hole 131 while flowing along the groove of the flow path 125 from both ends of the projection face 133. In this instance, a discharge amount of the liquid medicine transferred through the through hole 131 to the outflow hole OUTH is determined according to a position where the through hole 131 gets in contact with the flow path 125. If the purge portion 123 of the flow path 125 is located over the through hole 131, the liquid medicine fully filing the purge portion 123 is discharged to the outflow hole OUTH at once.

Differently from most of the conventional regulating apparatuses regulate the discharge amount of the liquid medicine by the inlet control method (the introduced amount of the liquid medicine is regulated while the flow path gets in contact with not the outlet but the inlet, and the discharged amount of the liquid medicine is regulated according to the introduced amount of the liquid medicine), the regulating apparatus 100 according to the present invention regulates the discharged amount of the liquid medicine while the through hole 131 of the projection face 133 connected with the outflow hole OUTH moves on the surface of the flow path 125, and hence, the regulating apparatus 100 regulates the flow rate of the liquid medicine by an outlet control method.

In case of most of the regulating apparatuses adopting the inlet control method, it is difficult to precisely regulate the flow rate of the liquid medicine because the resistance is strong from a standpoint of the liquid since the whole length of the flow path ranging from inflow to discharge of the liquid medicine. However, the regulating apparatus 100 according to the present invention can precisely regulate the flow rate of the liquid medicine because the resistance is weak from a standpoint of the liquid since the introduced liquid medicine fills the space and then is discharged when the flow path 125 gets in direct contact with the through hole 131.

Moreover, not shown in the drawings, but if the projection face 133 has a protrusion formed on one of both ends thereof to stop one side of the flow path, because the liquid medicine flows not from both sides of the projection face 133 but from just one direction, the user can regulate the discharged amount of the liquid medicine more precisely. Because the projection face 133 is made of rubber, if one end portion of the projection face 133 is projected a little using rubber, one side of the flow path 125 getting in contact with the projection face 133 can be naturally stopped.

Referring to FIGS. 7 and 8, the gate seal 130 includes: a surrounding portion 150 that is formed on the bottom face of the gate seal 130 in such a way as to be depressed upwardly and surrounds the second central hole CH2; and at least one fixing hole 151 formed in the outward direction of the bottom face from the surrounding portion 150.

Referring to FIG. 9, the insertion portion 177 of the body 170 includes: a fixing projection 179 that is formed on the bottom face of the insertion portion 177 and is inserted and fixed into the fixing hole 151 of the bottom face of the gate seal 130; and a third projection portion OT3 that surrounds the projection pole 171 and pressurizes the surrounding portion 150 of the bottom face of the gate seal 130 to fix the gate seal 130.

That is, the gate seal 130 is inserted into the insertion portion 177 of the body 170, and in this instance, the fixing projection 179 of the body 170 is inserted and joined into the fixing hole 151 of the gate seal 130 and the third projection portion OT3 of the gate seal 130 pressurizes the surrounding portion 150 of the gate seal 130, so that the gate seal 130 can be firmly fixed to the insertion portion 177 of the body 170.

The grip portion 180 of the body 170 is a portion that the user can grip with the hand, and the stopper 190 is mounted on the upper face of the grip portion 180 and inserted between the saw teeth of the first rotational gear portion G1 to fix the adjustment knob 110 so that the adjustment knob 110 is not rotated.

Referring to FIGS. 2 and 9, the stopper 190 includes: an upper body 195 that has a stopper handgrip 191 inserted between the saw teeth of the first rotational gear portion G1 and a detachably mounted portion 193 formed beneath the stopper handgrip 191; and a seating recess portion 197 that is formed integrally with the grip portion 180 and moves back and forth in a state where the detachably mounted portion 193 is forcedly inserted into the seating recess portion 197.

The upper body 195 has the stopper handgrip 191 and the detachably mounted portion 193. The stopper handgrip 191 includes: a sawteeth fixing portion 200 that is formed on the front end thereof and inserted between the saw teeth of the first rotational gear portion G1 to fix the first rotational gear portion 191 so that the first rotational gear portion G1 is not rotated; and a gradation check portion 210 formed above the sawteeth fixing portion 200 to allow the user to exactly see the gradations of the upper surface G2US of the second rotational gear portion G2. As shown in FIG. 1, the gradation check portion 210 has a hole formed at the center of a protruding front portion, so that the user can check the amount of the liquid medicine by means of the gradations shown through the hole.

As shown in FIG. 9, the seating recess portion 197 has a seating projection portion 199 protruding inwardly and having a U-shaped structure. The seating projection portion 199 prevents the detachably mounted portion 193 from getting out in a backward direction after the detachably mounted portion 193 is inserted into the seating recess portion 197, and the detachably mounted portion 193 is pressed and moved by the seating projection portion 199.

The regulating apparatus 100 having the above structure is easy in manufacturing because the number of the components is reduced and can precisely regulate the flow rate of the liquid medicine.

FIG. 11 is a view for explaining another structure of the gate seal.

As shown in FIG. 6, the projection face 133 protrudes upwardly from the upper face of the first tier 140. However, the gate seal 140 illustrated in FIG. 11 does not have the same structure as the projection face formed on the upper face of the first tier 140. A flow path groove 137 is formed along the upper face of the first tier 140 in such a way as to correspond to the flow path 125 of the bottom surface B1LS of the first body B1 and to be filled with the liquid medicine. In other words, the flow path groove 137 engraved on the upper face of the first tier 140 is formed at the position corresponding to the flow path 125.

The flow path groove 137 is formed in a round along the circumference of the first tier 140, but a start point and an end point of the flow path groove 137 do not meet with each other but are spaced apart from each other. Furthermore, the flow path groove 137 is connected with the inflow hole INH, so that the flow path groove 137 is filled with the liquid medicine introduced through the inflow hole INH.

The through hole 131 is formed between the start point and the end point of the flow path groove 137 in a fashion that the through hole 131 is spaced apart at a predetermined interval from the start point and the end point. When the upper face of the first tier 140 of the gate seal 130 gets in direct contact with the bottom surface B1LS of the first body B1, the flow path groove 137 and the flow path 125 are filled with the liquid medicine, and when the flow path 125 is located above the through hole 131 while the first body B1 is rotated, the liquid medicine is discharged through the through hole 131.

As described above, the example embodiment described in this specification is just the most preferred embodiment of the present invention. Terms and words used in the specification and claims are used to explain the particular embodiment, but there is no intent to limit the meanings of the terms and words or limit the scope of claims of the present invention to the terms and words.

It is to be appreciated that those skilled in the art can change or modify the embodiment without departing from the scope and spirit of the present invention. Accordingly, it will be also understood that the technical scope of the present invention should be determined by the technical idea of claims of the present invention.

### [Industrial Applicability]

The present invention is applicable to the field to manufacture apparatuses for injecting a liquid medicine.

## Claims

1. An apparatus for precisely regulating a liquid medicine injection dose comprising:
an adjustment knob having a first body having a flow path that is formed at a predetermined interval from the center of the bottom face thereof and has a depth that is different from a start point to an end point thereof, a second body being larger in diameter than the second body and formed integrally with the upper portion of the first body to thereby rotate with the first body, a third body being smaller in diameter than the first body and formed integrally with the lower portion of the first body to thereby rotate with the first body, and a first central hole being formed at the centers of the first body, the second body, and the third body;
a gate seal of a cylindrical shape being surrounding the first body and the third body, which are inserted into the gate seal, the gate seal including: an inflow hole formed in the wall surface of one side thereof for injecting the liquid medicine into the flow path; an outflow hole formed in the wall surface of the other side thereof for discharging the liquid medicine discharged from the flow path; a tiered portion formed along the inner wall surface and having a through hole that is formed in the upper face of the tiered portion, is in direct contact with a bottom surface of the first body and connected to the outflow hole, so that an amount of the liquid medicine is regulated by means of rotation of the flow path and the liquid medicine is discharged through the through hole; and a second central hole formed at the center of the tiered portion in correspondence to the first central hole; and
a body to which the gate seal is inserted and fixed, the body including: an insertion portion having a projection pole formed at the center of the bottom face and inserted into the first central hole and the second central hole to combine the adjustment knob with the gate seal, an inlet formed on one side of the wall surface for injecting the liquid medicine into the inflow hole, and an outlet formed on the other side of the wall surface in correspondence to the outflow hole; and a grip portion being elongated from the outer wall surface of the insertion portion in one direction.

2. The regulating apparatus according to claim 1, wherein the first body of the adjustment knob is formed in a cylindrical shape, and the flow path is formed in a concentric circle relative to the center of the bottom surface of the first body and comprises: a first portion which is gradually deepened from the start point to the end point; and a purge portion which is wider and deeper than the first portion but shorter than the first portion.

3. The regulating apparatus according to claim 2, wherein the second body of the adjustment knob comprises: a first rotational gear portion that is formed integrally to the upper portion of the first body, is larger in diameter than the first body, and has saw teeth formed on the circumferential surface thereof; and a second rotational gear portion that is formed integrally to the upper portion of the first rotational gear portion, is larger in diameter than the first rotational gear portion, and has saw teeth formed on the circumferential surface thereof and gradations formed on the upper surface for indicating the amount of the liquid medicine, and
Wherein the first rotational gear portion comprises: a first projection formed on the bottom surface thereof to prevent the liquid medicine from leaking out by pressing a portion which is in contact with the upper face of the wall surface of the gate seal; and a stop portion formed on the outer face of the first projection of the bottom surface of the first rotational gear portion and on the opposite side of the purge portion for preventing the second body rotating along the outer wall surface of the insertion portion of the body from rotating more.

4. The regulating apparatus according to claim 3, wherein a purge hole is formed in one side of the bottom face of the stop portion,
wherein a stop projection is protrudingly formed on the upper portion of the inlet of the outer wall surface of the body, so that the stop portion is caught to the stop projection and the second body is not rotated anymore,
wherein a purge projection is formed on the outer wall surface of the body spaced apart at a predetermined interval from the stop projection and coupled to the purge hole, so that the user can recognize the purge state that the amount of the liquid medicine is discharged to the maximum, and
wherein the purge projection is caught to the purge hole when the stop portion is caught to the stop projection.

5. The regulating apparatus according to claim 3, wherein a second projection portion is formed on the bottom surface of the third body for pressurizing a portion, which is in contact with the tiered portion of the gate seal to thereby prevent the liquid medicine from leaking, and
wherein a plurality of coupling poles are formed on the inner wall surface of the third body in which the first central hole is formed and respectively have coupling projections that respectively protrude at ends of the coupling poles toward the central direction, the coupling poles being upwardly projected in a cylindrical shape.

6. The regulating apparatus according to claim 3, wherein the tiered portion of the gate seal comprises:
a first tier formed along the inner wall surface of the gate seal and corresponding to the bottom surface of the first body;
a second tier formed beneath the first tier and along the inner wall surface of the first tier and corresponding to the bottom surface of the third body, the second tier having a diameter smaller than that of the first tier,
a projection face that has the through hole and is formed on the upper face of the first tier, the projection face projecting more upwardly than the remaining portion of the upper face of the first tier and being in direct contact with the bottom surface of the first body; and
a space formed between the remaining portion of the upper face of the first tier excepting the projection face and the bottom surface of the first body in such a fashion that the space is filled with the liquid medicine, and
wherein the through hole is formed vertically downward and is connected with the outflow hole at the bottom face of the gate seal.

7. The regulating apparatus according to claim 6, wherein the gate seal comprises:
a surrounding portion that is formed on the bottom face of the gate seal in such a way as to be depressed upwardly and surrounds the second central hole; and
at least one fixing hole that is formed in the outward direction of the bottom face from the surrounding portion.

8. The regulating apparatus according to claim 7, wherein the insertion portion of the body comprises:
a fixing projection that is formed on the bottom face of the insertion portion and is inserted and fixed into the fixing hole of the bottom face of the gate seal; and a third projection portion that surrounds the projection pole and pressurizes the surrounding portion of the bottom face of the gate seal to fix the gate seal,
wherein a stopper is mounted on the upper face of the grip portion and inserted between the saw teeth of the first rotational gear portion to fix the adjustment knob so that the adjustment knob is not rotated, and
wherein the stopper comprises: an upper body that has a stopper handgrip inserted between the saw teeth of the first rotational gear portion and a detachably mounted portion formed beneath the stopper handgrip; and a seating recess portion that is formed integrally with the grip portion and moves back and forth in a state where the detachably mounted portion is forcedly inserted into the seating recess portion.

9. The regulating apparatus according to claim 3, wherein the tiered portion of the gate seal comprises:
a first tier formed along the inner wall surface of the gate seal and corresponding to the bottom surface of the first body;
a second tier formed beneath the first tier and along the inner wall surface of the first tier and corresponding to the bottom surface of the third body, the second tier having a diameter smaller than that of the first tier, and
a flow path groove formed along the upper face of the first tier in such a way as to correspond to the flow path of the bottom surface of the first body and to be filled with the liquid medicine, and
wherein the through hole is formed between a start point and an end point of the flow path groove in such a fashion that the through hole is spaced apart from the start point and the end point of the flow path groove at a predetermined interval, and the liquid medicine filling the flow path groove and the flow path is discharged through the through hole when the upper face of the first tier gets in direct contact with the bottom surface of the first body, the flow path groove is connected with the inflow hole, and the through hole is formed vertically downward and connected with the outflow hole on the bottom face of the gate seal
